# EUROPEAN PATENT APPLICATION

(11) **EP 3 818 979 A1**
(43) Date of publication of application: **12.05.2021**
(21) Application number: 20206141.2
(22) Date of filing: 06.11.2020
(51) Int. Cl.: A61K 31/205, A61K 31/352, A61K 31/685, A61P 29/00

(54) **FOOD SUPPLEMENT WITH ANTIOXIDANT AND ANTI-INFLAMMATORY PROPERTIES**

(30) Priority: 08.11.2019 RO 201900718
(71) Applicant: Ionescu, John, 93485 Rimbach (DE); Zenyth Pharmaceuticals SRL, Judetul Neamt (RO)
(72) Inventor: IONESCU, John, 93485 Rimbach (DE)
(74) Representative: Teodorescu, Mihaela

(57) **Abstract**

The invention concerns a food supplement that contains a new mixture of natural components with antioxidant and anti-inflammatory properties, used for the control of oxidative stress and inflammation in human biologic systems.

The food supplement according of the invention is a mixture which comprises three or more of the following ingredients: carnosine, coenzyme Q10, phospholipids, pyrroloquinoline quinone (PQQ), fulvic acid, oligo-elements, vitamins.

It is conditioned for oral administration in the form of tablets, capsules, powder, or for intravenous administration in the form of infusion solutions which contains single dose for administration once or several times per day.

## Description

The present invention relates to a food supplement containing a new mixture of natural components having antioxidant and anti-inflammatory properties.
The food supplement according to the present invention has an antioxidant and free-radical-blocking ability that significantly exceeds the antioxidant activity of each individual component and, overall, has an effect of supporting the mitochondrial function.
The invention pertains to the pharmaceutical field.
Oxidative stress, inflammation, and metabolic dysfunction (mitochondrial dysfunction) lead to a gradual process of deterioration of the molecular balance, impairing the ability of the cell to fix or replace deteriorated molecular components, and results in greater sensitivity to diseases (Al Shahrani M. et al; Oxidative Stress: Mechanistic Insights into Inherited Mitochondrial Disorders and Parkinson's Disease. J Clin Med. 2017 Oct 27;6(11); Lenaz G, et al. Role of mitochondria in oxidative stress and aging. Ann N Y Acad Sci. 2002 Apr; 959:199-213; Barja G.; Free radicals and aging. Trends Neurosci. 2004 Oct;27(10):595-600.)
The body has a complex mechanism to protect itself against oxidation that preserves the balance between generation and degradation of reactive oxygen species (ROS) and reactive nitrogen species (RNS) through enzymatic and non-enzymatic endogenous and exogenous antioxidants (e.g. superoxide dismutase, catalase, peroxidase, glutathione, NADH, NADPH, thioredoxin and vitamins C and E, polyphenols, etc.) (Barja G.; Free radicals and aging. Trends Neurosci. 2004 Oct;27(10):595-600; Aruoma, Okezie I. "Free radicals, oxidative stress, and antioxidants in human health and disease." Journal of the American oil chemists' society 75.2 (1998): 199-212; Birben E, et al; Oxidative stress and antioxidant defense. World Allergy Organ J. 2012 Jan;5(1):9-19.)
If a deterioration of this balance leads to an accumulation of ROS, this can result in severe functional disorders. This set of conditions is named oxidative stress. The consequences of oxidative stress include lipid peroxidation, which, eventually, makes the cells to spend more energy to settle their membrane potential in order to counterbalance protein oxidation and DNA degradation (Wei YH. et al; Mitochondrial theory of aging matures--roles of mtDNA mutation and oxidative stress in human aging. Zhonghua Yi Xue Za Zhi (Taipei). 2001 May;64(5):259-70). These three processes are responsible for the aging process, and they can cause a wide range of disorders, such as cardiovascular diseases, diabetes, glaucoma, hearing loss, infertility and Parkinson's disease, Alzheimer's disease, etc. (Liochev SI.; Reactive oxygen species and the free radical theory of aging. Free Radic Biol Med. 2013 Jul; 60:1-4; Jenner, Peter. "Oxidative stress in Parkinson's disease." Annals of Neurology: Official Journal of the American Neurological Association and the Child Neurology Society 53.S3 (2003): S26-S38; Maritim, A. C., RA Sanders, and J. B. Watkins III. "Diabetes, oxidative stress, and antioxidants: a review." Journal of biochemical and molecular toxicology 17.1 (2003): 24-38)
The term "reactive oxygen species" (ROS) defines oxygen metabolites and oxygen radicals resulting as by-products of normal aerobe metabolism and includes non-radical molecules (hydrogen peroxide = H₂O₂), as well as radical molecules (free radicals), for instance the hydroxyl radical (•OH), superoxyde anion radical (O₂•-), singlet oxygen (¹O₂), hypochlorite (HClO-), nitric oxyde radical (•NO) and peroxynitrite radical (ONOO^{•}).
ROS and RNS have specific physiological activity. They eliminate intra- and extracellular germs, participate in regulating the metabolism, control of enzymatic reactions, and regulate gene expression through redox-sensitive transcription factors but they may also have toxic effects that might result in sub-cellular and cellular degradation, causing various pathologies (Hancock, J. T., R. Desikan, and S. J. Neill. "Role of reactive oxygen species in cell signalling pathways." Biochemical Society Transactions 29.2 (2001): 345-349; Davies KJ; Oxidative stress: the paradox of aerobic life. Biochem Soc Symp. 1995; 61:1-31).
These extremely reactive species can impair biologically relevant molecules such as DNA, proteins, carbohydrates and lipids, and lead to the degradation of cell membranes and homoeostatic disruption (Berlett, Barbara S., and Earl R. Stadtman. "Protein oxidation in aging, disease, and oxidative stress." Journal of Biological Chemistry 272.33 (1997): 20313-20316).
Chronic inflammation plays a central role in the evolution of the aging process and the onset of age-related diseases, both through the cumulative effect of environmental toxins (metals, pesticides, solvents, phthalates, etc.) and through increasng sensitivity to chronic bacterial, viral or mycotic infections. The immune system becomes increasingly dysfunctional, mainly through an imbalance of the redox status. The change of the redox status in favor of a pro-oxidative micro-environment is caused both by a decrease in the activity of antioxidant enzymes and a deficiency of external antioxidants, that leads to an increasing production of ROS and RNS, followed by a harmful activation of the immune system through an over-activation of macrophage cells during the inflammatory processes. (Berlett, Barbara S., and Earl R. Stadtman. "Protein oxidation in aging, disease, and oxidative stress." Journal of Biological Chemistry 272.33 (1997): 20313-20316; Davies KJ; Oxidative stress, antioxidant defenses, and damage removal, repair, and replacement systems. IUBMB Life. 2000 Oct-Nov;50(4-5):279-89; Wei YH. et al; Oxidative stress in human aging and mitochondrial disease-consequences of defective mitochondrial respiration and impaired antioxidant enzyme system; Chin J Physiol. 2001 Mar 31;44(1):1-11).
The aging process is associated with an increase of free radicals, thromboxane A2, and an increased production of prostacycline, catalyzed by cyclooxygenase enzymes, as well as an over-regulation of numerous inflammatory genes and proteins, including IL-1b, IL-6, TNF-a, iNOS, Cox-2 and different adhesion molecules (ICAM-1, VCAM-1, P-selectine, and E-selectine). NF-kB, thought to be a key regulator of the inflammatory process, is activated through free radicals in a pro-oxidative environment, and it has been proved to be involved in a wide range of systemic inflammatory conditions such as Alzheimer's disease, cancer, multiple sclerosis, diabetes and cardiovascular diseases (Evans, P. H. "Free radicals in brain metabolism and pathology." British medical bulletin 49.3 (1993): 577-587; Fearon, Ian M., and Stephen P. Faux. "Oxidative stress and cardiovascular disease: novel tools give (free) radical insight." Journal of molecular and cellular cardiology 47.3 (2009): 372-381).
Mitochondria is responsible for the generation of adenosine triphosphate (ATP) through a chain of processes called "oxidative phosphorylation", for orchestrating various cellular processes, including cell differentiation, death of cells, and regulation of cell cycle. Although ATP generation is of critical importance in supplying of many cell processes, the ROS production as a natural by-product of the oxidative phosphorylation is both a benefit and a challenge. Even though, in moderate amounts, ROS can serve as efficient signal molecules for inducing protective responses to the cellular stress (Cadenas E, et al; Mitochondrial free radical generation, oxidative stress, and aging. Free Radic Biol Med. 2000 Aug;29(3-4):222-30; Duberley KE, et al; Human neuronal coenzyme Q10 deficiency results in global loss of mitochondrial respiratory chain activity, increased mitochondrial oxidative stress and reversal of ATP synthase activity: implications for pathogenesis and treatment. J Inherit Metab Dis. 2013 Jan;36(1):63-73), an excessive release of electrons from the complexes I and III of the electron transport chain (ETC) and further formation of superoxide and hydrogen peroxide radicals causes oxidative damage, including deterioration of mitochondrial and nuclear DNA (leading to transmissible mutations) and shortening of length of telomeres, and acceleration of the body aging process. Further blocking of DNA transcription and replication can impair several metabolic activities occurring in mitochondria, such as ATP generation, amino acid metabolism and oxidation of fatty acids (Evans, P. H. "Free radicals in brain metabolism and pathology." British medical bulletin 49.3 (1993): 577-587).
Beside metabolic disruptions, other harmful effects of oxidative mitochondrial degradation include inappropriate activation of apoptosis through permeabilization of the outer mitochondrial membrane and subsequent release of cytochrome c in the cytosol and induction of transition pores of mitochondrial permeability (which results in permeabilization of the inner membrane in pathological conditions such as a stroke or cerebral injury). Many conditions are characterized by defects of cell metabolism and oxidative phosphorylation (diabetes, hypertension, Parkinson's disease, glaucoma and age-related macular degeneration) (Stepien KM. et al: Evidence of Oxidative Stress and Secondary Mitochondrial Dysfunction in Metabolic and Non-Metabolic Disorders. J Clin Med. 2017 Jul 19;6(7); Berlett, Barbara S., and Earl R. Stadtman. "Protein oxidation in aging, disease, and oxidative stress." Journal of Biological Chemistry 272.33 (1997): 20313-20316)
Oxidative stress, inflammation and metabolic degradation are the causes of many pathologies age-related or not.
Various options of symptomatic treatment are known for these pathologies, usually focused on medicines designed to work against the pathophysiology of the disease, but not the cause thereof.
In many cases, these medicines exhaust the number of mitochondria and lead to an increase of ROS in the mitochondria, to slowing down of the phosphorylation process, to a decrease of ATP production and to compromising integrity of the cell and mitochondrial membrane.

Within this context, the technical problem addressed by the present invention consists of a systemic, causal approach, by creating a food supplement that reduces systemic oxidative stress and chronic inflammation and improves mitochondrial biogenesis and the functionality of mitochondria.
The food supplement according to the invention provides a new nutritional composition, that comprises three or more antioxidant components, as well as sublayers, co-factors and modulators with enzymatic activity.
The technical solution to this problem consists of an association of some components, in the form of a food supplement having antioxidant and anti-inflammatory properties, for the use to control the oxidative stress and inflammation in human biological systems, which is a mixture which comprises three or more of the following ingredients: carnosine, coenzyme Q10, phospholipids, pyrroloquinoline quinone (PQQ), fulvic acid, oligo-elements, vitamins.
The food supplement according to the invention has remarkable antioxidant and anti-inflammatory properties, with applicability in the treatment/management of skin disorders, autoimmune diseases, diabetes, rheumatic diseases, chronic fatigue syndrome (CFS), cardiovascular diseases, and neurodegenerative conditions (Parkinson's, Alzheimer's).
Also, another result of the application of the invention is the prevention of nutritional deficiencies and conditions caused by the harmful effects of oxidative stress, caused by free radicals (cardiovascular diseases, cancer, degenerative conditions, autoimmune diseases, etc.).
The results clearly show a strong and fast response of the combination when given orally, as well as the absence of any kind of secondary effects. After several days of treatment, the patients report a significant alleviation of fatigue, headache, lack of concentration, diffuse muscular pain (fibromyalgia) and arrhythmia symptoms.
Further on, it is a brief presentation of the figures that are part of the invention:
Figure 1A - shows the results of the antioxidant activity of the individual components
Figure 1B - shows the results regarding the antioxidant activity of 3 combinations of ingredients
Figure 2 - shows the antioxidant effect of the mixture C on the production of free radicals in whole blood ex *in vivo*
Figure 3A - shows the elimination of free radicals from blood plasma samples after the addition of 1% solution of combination C
Figure 3B - shows elimination of free radicals from blood plasma samples after the addition of 4% solution of combination C
Figure 4 - highlights the significant decrease of free radicals in the whole blood of a patient suffering from generalized atopic neurodermatitis after administration of two capsules of combination C
Figure 5 - highlights the increase of antioxidant total ability of blood plasma one hour after administration of two capsules of combination C
Figure 6 - highlights the effect of usual dose of combination C on the production of free radicals after 3 days of administration in a patient with chronic fatigue syndrome (CFS), recurrent viral infections and fibromyalgia.
In one embodiment of the invention, the food supplement is a mixture which comprises coenzyme Q10, PQQ, and fulvic acid.
In another embodiment of the invention, the food supplement is a mixture which comprises L-carnosine, magnesium phosphate, potassium phosphate, coenzyme Q10, phosphatidylcholine, and PQQ.
In another embodiment of the invention, the food supplement is a mixture which comprises L-carnosine, magnesium phosphate, potassium phosphate, coenzyme Q10, phosphatidylcholine, silicone dioxide 1.5-10.0%; PQQ, fulvic acid, folic acid and vitamin B12.The synergic action of the components of the mixture ensure much higher antioxidant and anti-inflammatory activity compared to the activity of each individual component taken separately.
The present invention provides a new nutritional composition that comprises carnosine, coenzyme CoQ10, pyrroloquinoline quinone (PQQ), phosphatidylcholine, folic acid, vitamin B12, fulvic acid, potassium and magnesium salts of phosphoric acid. All components work together in synergy and they were selected in order to eliminate antagonistic interactions between them. In other words, the food supplement is designed to ensure a high compatibility between the components, and total antioxidant activity at least 500% higher than that of its components, assessed individually.
Carnosine (C9 H14 N4 O3), also known as N-β-Alanyl-L-histidine, is a dipeptide composed of beta-alanine and histidine amino acids, accounting for much of the intracellular nitrogen stocks and serving as a stock for histidine (a precursor of histamine, produced during stress)

Carnosine was proven to eliminate reactive oxygen species (ROS), as well as unsaturated alpha-beta aldehydes resulted from peroxidation of fatty acids of the cell membrane during oxidative stress, it has anti-inflammatory, antioxidant and anti-glicant properties and chelates divalent metal ions (Sandra Budzeń et al; The Biological Role of Carnosine and Its Possible Applications in Medicine; Adv Clin Exp Med 2013, 22, 5, 739-744; Tsai, Shih-Jei, et al. "Antioxidative and anti-inflammatory protection from carnosine in the striatum of MPTP-treated mice." Journal of agricultural and food chemistry 58.21 (2010): 11510-11516; Fouad, Amr A., et al. "Protective role of carnosine in mice with cadmium-induced acute hepatotoxicity." Food and chemical toxicology 47.11 (2009): 2863-2870).
Due to these properties, L-carnosine counteracts accelerated aging and genome degradation (supports cell integrity and functionality).
Carnosine binds to the carbonyl/aldehyde groups resulted from oxidative degradation of reductive sugars (Maillard reaction), it prevents or reverses their coupling to proteins, that protects against deterioration and against formation of final glycoxidation products (AGE) (Tsai, Shih-Jei, et al. "Antioxidative and anti-inflammatory protection from carnosine in the striatum of MPTP-treated mice." Journal of agricultural and food chemistry 58.21 (2010): 11510-11516). It is also a powerful inhibitor of lipid peroxide formation and end products of lipid oxidation (ALE) (Aldini G, Facino RM, Beretta G, Carini M. Carnosine and related dipeptides as quenchers of reactive carbonyl species: from structural studies to therapeutic perspectives. Biofactors 24: 77- 87, 2005). It also promotes vimentin production (compound with important role in the elimination of oxidized proteins that contribute to aging) and, through chelation and elimination of heavy metals, it contributes in decreasing of lipid, DNA and protein degradation process (Boldyrev AA, et.al;. Carnosine as a natural antioxidant and geroprotector: From molecular mechanisms to clinical trials. Rejuvenation Res. 2010; 13:156).
It has been found that carnosine has a double ability to influence the immune response: it decreases excessive immune response in patients with a hyperactive immune system, while increasing immune response in patients with an under-active immune system (such as elderly persons), which makes it a vital tool for people having a sensitive immune system, such as people suffering from allergies and autoimmune diseases (Salah E. et al; Carnosine: physiological properties and therapeutic potential. Age and ageing 2000, 29: 207- 210).

The antitumor properties of carnosine have been recognized for more than three decades ago and consist in reducing DNA destruction (that contributes in changing healthy cells into cancer cells), in inhibiting tumor growth and in preventing metastases, in depriving cancer cells from energy by decreasing the ATP level just in these cells (Gaunitz, Frank, and Alan R. Hipkiss. "Carnosine and cancer: a perspective." (2012): 135-142).
Coenzyme Q10 (CoQ10) (2,3-dimethoxy-5-methyl-6-decaprenil benzoquinone) is a natural substance soluble in fats that plays a main role as an enzyme cofactor necessary in the transporting chain of mitochondrial electrons, and it is key factor in oxidative phosphorylation and aerobe cell breathing by generating adenosine triphosphate (ATP) (Egil Fosslien; Mitochondrial Medicine - Molecular Pathology of Defective Oxidative Phosphorylation, Annals of Clinical and Laboratory Science, 2000).
CoQ10 was proven to act both as an antioxidant and as an electron carrier due to its lipid solubility and its capacity to exist in both completely reduced forms (ubiquinol) and completely oxidized forms (ubiquinone).
CoQ10 accepts electrons derived from both complex I (NADH ubiquinone oxidoreductase) and complex II (succinate ubiquinone reductase), and then transports them to complex III (ubiquinol cytochrome c reductase).
CoQ deficiency (associated with inadequate food intake or a disruption of the mechanism of CoQ production due to inhibition of endogenous cholesterol production following statine administration) may have an impact on the electron transport in the mitochondrial breathing chain, with proliferation of oxygen radicals, leading to increase of oxidative stress and, eventually, to intensification of lipid peroxidation (Sohal RS., et al. Effect of coenzyme Q10 intake on endogenous coenzyme Q content, mitochondrial electron transport chain, antioxidant defenses, and life span of mice. Free Radic Biol Med. 2006;40(3):480-487).
The antioxidant properties of CoQ10 indicate that it may be efficient in treating several diseases or conditions associated with increased oxidative stress (cardiovascular diseases, cancer, neurodegenerative diseases such as Parkinson's and Alzheimer's) (Lee BJ, et al. Coenzyme Q10 supplementation reduces oxidative stress and increases antioxidant enzyme activity in patients with coronary artery disease. Nutrition 2012;28(3):250-255; Shults, Clifford W., et al. "Effects of coenzyme Q10 in early Parkinson disease: evidence of slowing of the functional decline." Archives of neurology 59.10 (2002): 1541-1550; Dai YL, et al. Reversal of mitochondrial dysfunction by coenzyme Q10 supplement improves endothelial function in patients with ischemic left ventricular systolic dysfunction: a randomized controlled trial. Atherosclerosis. 2011 Jun;216(2):395-401; Limón-Pacheco J. et al; The role of antioxidants and antioxidant-related enzymes in protective responses to environmentally induced oxidative stress. Mutat Res. 2009 Mar 31;674(1-2):137-47; Wadsworth TL, et al. Evaluation of coenzyme Q as an antioxidant strategy for Alzheimer's disease. J Alzheimers Dis. 2008 Jun;14(2):225-34; Fan L, et al.; Effects of coenzyme Q10 supplementation on inflammatory markers: A systematic review and meta-analysis of randomized controlled trials. Pharmacol Res. 2017; 119:128-136).
Pyrroloquinoline quinone (PQQ), found in 1979, is a key co-factor in the redox cycle, acting directly as an essential nutrient on the key enzymes of mitochondria, facilitating energy production and mitochondrial biogenesis (spontaneous generation of new mitochondria in aged cells) (He K, et. al; . Antioxidant and pro- oxidant properties of pyrroloquinoline quinone (PQQ): implications for its function in biological systems. Biochem. Pharmacol. 2003; 65:67-74; Calliandra B. et al; Dietary pyrroloquinoline quinone (PQQ) alters indicators of inflammation and mitochondrial- related metabolism in human subjects; The Journal of Nutritional Biochemistry; Volume 24, Issue 12, December 2013, Pages 2076-2084).
PQQ increases the ability of mitochondria to produce and store adenosine triphosphate; it activates some molecular signaling paths that result in a stimulation of the formation of new mitochondria in aged cells, and enhances the activity of genes that support mitochondrial reproduction, protection and repair (Stites T, et al. Pyrroloquinoline quinone modulates mitochondrial quantity and function in mice. J Nutr. 2006 Feb;136(2):390-6; Chowanadisai W et.al.; Pyrroloquinoline quinone stimulates mitochondrial biogenesis through cAMP response element-binding protein phosphorylation and increased PGC- 1alpha expression; J Biol Chem. 2010 Jan 1;285(1):142-52).
In pre-clinical trials, when animals deprived of dietary PQQ (these animals displayed an irregular increase, with a low conception rate and low mitochondria counts) were given a PQQ diet, it was found reversing the effects, while increasing mitochondria counts and increasing energy efficiency (Misra HS, et al; Pyrroloquinoline-quinone and its versatile roles in biological processes. J Biosci. 2012 Jun;37(2):313-25).
Based on said trials it has been proved that PQQ, both by itself and combined with CoQ10, improves and supports heart health and brain functions (Ohwada K, et al. Pyrroloquinoline quinone (PQQ) prevents cognitive deficit caused by oxidative stress in rats. J. Clin. Biochem. Nutr. 2008; 42:29-34).
In the brain, the main effect of PQQ is neuroprotection (through the redox mechanism and activation of the Nrf2 factor) and nerve growth (neurogenesis) (Zhang Q, et.al.; Pyrroloquinoline quinone rescues hippocampal neurons from glutamate-induced cell death through activation of Nrf2 and up-regulation of antioxidant genes. Genet Mol Res. 2012 Aug 16;11(3):2652-64).
A study on PQQ showed a 40 times increase of the nerve growth factor (NGF) and a 20-30% increase of mitochondria counts in cell cultures (Chowanadisai W et.al.; Pyrroloquinoline quinone stimulates mitochondrial biogenesis through cAMP response element-binding protein phosphorylation and increased PGC- 1alpha expression; J Biol Chem. 2010 Jan 1;285(1):142-52).
As for its antioxidant activity, PQQ proved to be particularly efficient in neutralization of two of the strongest free radicals, superoxide and hydroxyl radicals (Tao, Rong, et al. "Pyrroloquinoline quinone preserves mitochondrial function and prevents oxidative injury in adult rat cardiac myocytes." Biochemical and biophysical research communications 363.2 (2007): 257-262; Urakami, Teizi, et al. "Synthesis of monoesters of pyrroloquinoline quinone and imidazopyrroloquinoline, and radical scavenging activities using electron spin resonance in vitro and pharmacological activity in vivo." Journal of nutritional science and vitaminology 43.1 (1997): 19-33), while also inhibiting peroxynitrite formation (Zhang, Yumin, and Paul A. Rosenberg. "The essential nutrient pyrroloquinoline quinone may act as a neuroprotectant by suppressing peroxynitrite formation." European Journal of Neuroscience 16.6 (2002): 1015-1024).
Based on trials on animals, the researchers investigating the impact of PQQ on cardiovascular disease proved that administration thereof reduces the acute myocardial infarct size and favorably reduces lipid peroxidation, helps heart muscle cells to deal with acute oxidative stress, preserves and improves the mitochondrial function (Bauerly K et.al; Altering pyrroloquinoline quinone nutritional status modulates mitochondrial, lipid, and energy metabolism in rats; PLoS One. 2011;6(7); Zhu, Boqing, et al. "Pyrroloquinoline quinone (PQQ) decreases myocardial infarct size and improves cardiac function in rat models of ischemia and ischemia/reperfusion." Cardiovascular drugs and therapy 18.6 (2004): 421-431).
The fulvic acid is the main component of shilajit - a phyto-mineral substance (originating from Himalaya). It stabilizes, revitalizes and preserves CoQ10 in its active form (ubiquinol), and stimulates available CoQ10 levels to protect against mitochondrial aging, while handling the transport of nutrients to body tissue.
The components of shilajit, including fulvic acid, serve as an electron reservoir which compensates the electrons lost by CoQ10 during the redox reaction, and allow this vital coenzyme to remain active for a longer time (Agarwal SP, et al. Shilajit: a review. Phytother Res. 2007 May;21 (5):401-5; Visser SA.; Effect of humic substances on mitochondrial respiration and oxidative phosphorylation. Sci Total Environ. 1987 Apr; 62:347-54).
In studies on laboratory animals, the shilajit and CoQ10 combination led to an increase of ATP production by 27% in muscle cells and 40% in brain cells, compared to the administration of CoQ10 alone. Fulvic acid supports CoQ10 and electron transfer in mitochondria and stimulates mitochondrial energy metabolism, protecting mitochondria membranes from oxidative damages (Bhattacharyya S, et al. Shilajit dibenzo- α-pyrones: Mitochondria targeted antioxidants.Pharmacologyonline. 2009; 2:690-8; John Winkler, et.al.; Therapeutic Potential of Fulvic Acid in Chronic Inflammatory Diseases and Diabetes; J Diabetes Res. 2018; Goel, R. K., R. S. Banerjee, and S. B. Acharya. "Antiulcerogenic and antiinflammatory studies with shilajit." Journal of Ethnopharmacology 29.1 (1990): 95-103).
Phosphatidylcholine is a natural phospholipid (a component of lecithin) that is a major source of choline (the main lipid component of cell membranes and blood proteins), a nutrient that acts as a precursor of acetylcholine neurotransmitter (a vital substance for cell membranes, for functioning of memory, nervous system and muscles, which supports the communication between nerve cells and enables better transmission of nerve influx in the brain and maintains health of the myelin sheath of the neurons).
Phosphatidylcholine is necessary in composing and repairing cell membranes of the body, and it is vital for the liver function. Recent studies reported the antioxidant and anti-inflammatory activity of phosphatidylcholine (Meijuan Chen,et. al.; Phosphatidylcholine regulates NF-κB activation in attenuation of LPS-induced inflammation: evidence from in vitro study; Molecular & cellular biology, 2017, Pages 7- 14).
Folic acid (vitamin B9 or folate) is essential in the development and functioning of the body, due to its key role in cell growth and regeneration, plays role of a coenzyme for neurotransmitters, and at the same time it is involved in the synthesis of nucleic acids, thus ensuring proper functioning of the nervous system (Lubna Mahmood; The metabolic processes of folic acid and Vitamin B12 deficiency; Journal of health research and reviews. 2014, 1: 5-9).
5-Methyltetrahydrofolate takes part to the homocysteine metabolism and prevents cardiovascular diseases.
Vitamin B12 has a key role in DNA synthesis during cell division, for hemoglobin synthesis (substance accounting for oxygen transport in blood), for normal lipid and protein metabolism (plays an active role in fatty acid synthesis and fat burning in the body), as well as for energy synthesis in the body.
Vitamin B12 takes an active part also in cell renewal, and it is used in restoring the active form of folic acid as methylfolate. Methylfolate is a reaction product in homocysteine methylation into methionine in DNA synthesis, and it is also important in cardiovascular protection (Michael Fenech; Folate (vitamin B9) and Vitamin B12 and their function in the maintenance of nuclear and mitochondrial genome integrity; Mutation Research/Fundamental and Molecular Mechanisms of Mutagenesis 733(1-2):21-33).
Magnesium, as an essential mineral and electrolyte for the human body, has more than 300 functions in the body and plays a crucial role in hormonal balance (e.g. thyroid function, estrogen detoxification, blood sugar, stress hormones, etc.). Localized mainly inside the cells, magnesium contributes to ensure the transport of electrolytes (potassium, calcium) and other nutrients through the cell membranes, regulating blood sugar and maintaining the health of the cardiovascular system (Dyckner, Thomas, and Per Ola Wester. "Potassium/magnesium depletion in patients with cardiovascular disease." The American journal of medicine 82.3 (1987): 11-17). Magnesium balances the pH in the body and supports proper enzyme action, creates energy by activating adenosine triphosphate (ATP) synthesis, and helps in DNA replication (Elisabeth Gout et. al; Interplay of Mg2+, ADP, and ATP in the cytosol and mitochondria: Unravelling the role of Mg2+ in cell respiration; PNAS | October 13, 2014).

Potassium is a vital mineral for the functioning of nerves and muscles and for maintaining of proper heart rhythm. It helps to control of sodium growing effects on blood pressure and to relax the smooth muscles in the blood vessel walls. Potassium ions are of great importance for the electric membrane balance and the functioning of nerve and muscle cells, as well as for the acid-base balance (D'Elia, Lanfranco, et al. "Potassium intake, stroke, and cardiovascular disease: a meta-analysis of prospective studies." Journal of the American College of Cardiology 57.10 (2011): 1210-1219).
Phosphorus is an important mineral element and integral part of nucleic acids in DNA (deoxyribonucleic acid), takes part to the structure thereof having a key function in storing and generating energy. At the same time it is a buffer in the blood acid-base balance helping in stabilizing pH values in the blood (Reimer, K. A., R. B. Jennings, and M. L. Hill. "Total ischemia in dog hearts, in vitro 2. High energy phosphate depletion and associated defects in energy metabolism, cell volume regulation, and sarcolemmal integrity." Circulation research 49.4 (1981): 901-911; Coty, William A., and Peter L. Pedersen. "Phosphate Transport in Rat Liver Mitochondria Kinetics and Energy Requirements." Journal of Biological Chemistry 249.8 (1974): 2593-2598).
The food supplement according to the invention is prepared using methods known to a person skilled in the art.

The food supplement mixture is made for oral administration, in the form of tablets, capsules, powder or for intravenous administration in the form of infusion solutions which contains single dose for administration once or several times per day.

Further on it is provided a preparation example (C).

In order to prepare a capsule of food supplement the following ingredients are weighed: 22 g folic acid, 26 g B12 vitamin, 71 g fulvic acid (50%) obtained from Shilajit and are dispersed through homogenization in a dry powder mixer together with 4.412 kg carnosine, for 20 minutes.

Separately, 1.765 kg ubiquinone, 0.176 Kg PQQ, 1.588 kg phosphatidylcholine (20-30% obtained from sunflower lecithin, soy, or egg yolk), and 0.375 Kg silicone dioxide, are weighed and homogenized in a mixer for 15 minutes.

4.165 kg magnesium phosphate and 2.4 kg potassium phosphate are weighed and on these there are added to the previously obtained two mixtures. This mixture is blended for 15 minutes.

The obtained homogeneous mixture is packed in type 000 capsules, each capsule having an average weight of 850 mg.

Each capsule thus obtained contains: 250 mg carnosine, 100 mg CoQ10, 30 mg phosphatidylcholine, 10 mg PQQ, 2 mg fulvic acid, 1500 micrograms vitamin B12, 1250 micrograms folic acid, 95.1 mg phosphorus, 35.9 mg potassium and 50.0 mg magnesium.

The recommended dose is 2-4 capsules/day.

The food supplement according to the invention was assessed for antioxidant activity.

All living organisms have a very weak photon emissions comprised in the range of 180-800 nm of the light spectrum and this phenomenon is closely related to the oxidative metabolism of free radical production, cell division, cell apoptosis, photosynthesis, premature aging, cancer genesis and growth regulation, and in specific instances, that stimulate phagocytosis and activation of NADPH oxidase of polymorphonuclear leucocytes (PMN), there is a photon emission, measurable through scintillation counter. Various methods for measuring the antioxidant activity have been developed in the past decades (AOA) of biological solutions and components. Some of these use a source for producing free radicals, a chemiluminescence (CL) buster (e.g. luminol, lucigenin), a powerful oxidant (e.g. perborate), enzymatic catalysts such as peroxidase, xanthine oxidase, and busters such as para iodophenol. During oxidation, luminol and lucigenin release light energy when excited electrons fall back into their normal state. Continuous light emission mirrors the production of reactive O₂ (superoxide, hydrogen peroxide, hydroxyl), and has a sensitive reaction to interruptions induced by free radical scavengers (Ionescu G. et al.: Simple chemiluminescence assays for free radicals in venous blood and serum samples. Results in atopic, psoriasis, MCS and cancer patients. Forschende Komplementärmedizin, 6, 294-300, 1999).

The 3-steps CL method consists in the addition to a constant amount of blood, serum or antioxidant solution (e.g. fruit juice, a mixture of antioxidants, etc.), of a constant amount of buffered lucigenin (in the AOA test, a ROS-producing mixture), followed by a brief pre-incubation and then measuring the number of photons at 600-second intervals, at body or room temperature (37°C, respectively 22°C) (Ionescu G. et al.: Simple chemiluminescence assays for free radicals in venous blood and serum samples. Results in atopic, psoriasis, MCS and cancer patients. Forschende Komplementärmedizin, 6, 294-300, 1999; lonescu J.G. et al.: Clinical Application of Free Radical Assessment in Blood and Serum Samples by Enhanced Chemiluminescence. II. Antioxidative activity and therapy approaches with drugs and natural compounds. J. Biomed. Lab. Sci, 12, 46-56, 2000; lonescu J.G.: Klinische Relevanz der Redox- und Chimioluminiszenzbestimmungen bei Allergien, Haut- und Umwelterkrankungen. In: Marktl, Reiter, Ekmekcioglu: Säuren, Basen, Schlacken, Springer-Verlag, 73-81, 2007).

Deviations of the described parameters (occurrence of a high number of free radicals in cell compartments, respectively modifications of redox values), as well as of other immunologic parameters were already described in psoriasis, neurodermatitis and multiple chemical sensitivity (MCS), these being the consequence of inappropriate nutrition, persistent infections and harmful substances (Ionescu JG: Free radical monitoring in human blood following therapy interventions with drugs and natural compounds. Médecine & Longévité 2010; 2(4): 211-220; lonescu J.G.: Integrative Psoriasistherapie unter Berücksichtigung der Provokationsfaktoren. Derm 11, 59-64, 2005; lonescu JG: New insights in the pathogenesis of atopic disease. Journal of Medicine and Life 2009; 2(2) 146-154).

The assays conducted in the laboratories of the "Neukirchen Special Clinic" in Germany (http://www.spezialklinik-neukirchen.de/) show the superiority of mixture (C) in terms of antioxidant capacity ("free radical quenching capacity") both in respect of the components of the mixture taken individually as well as in respect of some combinations of ingredients (A, B) tested in parallel.

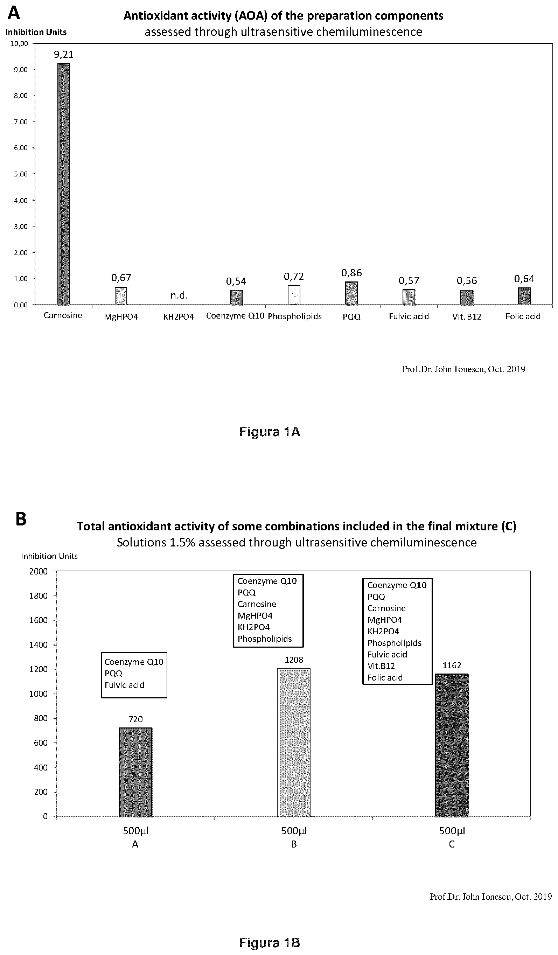

Figure 1A shows the results of antioxidant activity of the individual components, while Figure 1B shows the results of antioxidant activity of 3 combinations of ingredients, namely combination A (Coenzyme Q10, PQQ, and fulvic acid), combination B (Coenzyme Q10, PQQ, carnosine, Mg salt, K salt, phospholipids), and combination C (Coenzyme Q10, PQQ, carnosine, Mg salt, K salt, phospholipids, fulvic acid, vitamin B12 and folic acid). Both combinations A and B and combination C (250 mg carnosine, 100 mg CoQ10, 30 mg phosphatidylcholine, 10 mg PQQ, 2 mg fulvic acid, 1500 micrograms vitamin B12, 1250 micrograms folic acid, 95.1 mg phosphorus, 35.9 mg potassium, 50.0 mg magnesium) are tested as solutions of 1.5% concentration.

The results show:
1) Testing the antioxidant capacity of individual components of the mixture, as concentrations in the final product, shows insignificant antioxidant activity (below unit), except for carnosine (9.21 AOA inhibition units). (figure 1A)
2) Tested combinations A, B, C show particularly significant increases and clinically relevant of antioxidant and anti-inflammatory activity, proven through examples (A, B and C). (figure 1B)
3) Highly significant effects and clinically relevant are noticed for combination B and combination C by association of carnosine, phospholipids, magnesium and potassium salts, folic acid and vitamin B12.

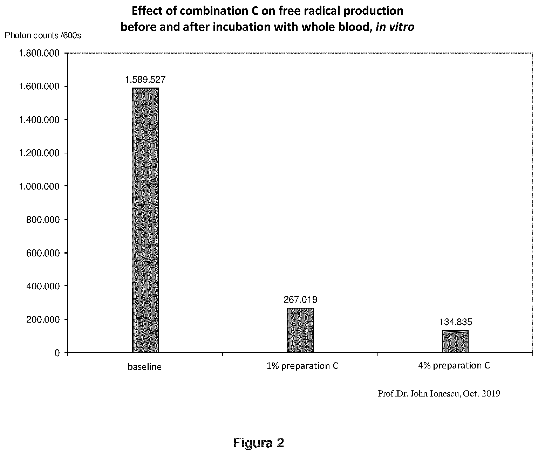

The food supplement according to the invention was further assessed through tests on ex in vivo blood and plasma samples collected from patients with exacerbated inflammation documented through extremely high values of free radicals (atopic neurodermatitis). (figure 2)

The free radical production was measured using the ultrasensitive chemiluminescence test in a fresh sample of 500 µl of blood collected from a patient with acute atopic neurodermatitis (baseline value). The result of 1,589,527 photons counted in 600 seconds proves an extremely powerful inflammatory condition.

500 µl of the same blood sample were incubated in 500µl of a 1% solution of the preparation. The result shows an outstanding decrease of free radicals to 267,019 counted photons.

500 µl of the same blood sample were incubated in 500µl of a 4% solution of the preparation. The result shows an outstanding decrease of free radicals to 134,835 counted photons.

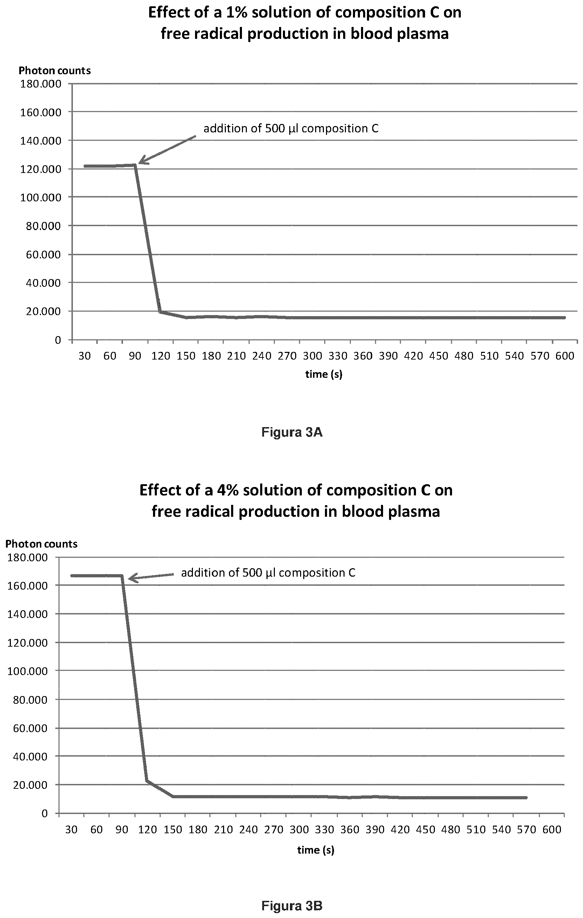

The assessment of the antioxidant effect of the food supplement was also tested in plasma collected from the same patient, obtained through blood centrifugation. After 90 second incubation/conditioning of a plasma/lucigenin mixture, 500µl of a 1% or 4% solution (arrow) of combination C, was added, and counting photon emission continued. After adding the solution of preparation C, a dramatic decrease of the number of counted photons and therefore, of free radicals in the sample is noted (figure 3A and figure 3B).

The results reveal that the mixture of components used in combination C show extremely powerful antioxidant activity in assays with fresh ex in vivo human blood.

The food supplement according to the invention was tested *in vivo* for its antioxidant effect on the production of free radicals in whole blood after oral administration.

To evaluate the *in vivo* effect of the subject matter of the invention, two capsules of 750 mg of preparation C were given, in fasting state, to a patient with generalized atopic neurodermatitis characterized by strong systemic inflammation.

The test assessed (a) the dynamics of free radicals decrease before administration, at one hour and, respectively, at 3 hours after administration of the preparation, and (b) the effect of the preparation on the antioxidant activity (AOA) in plasma, also, before administration, at one hour and at 3 hours after administration of the preparation.

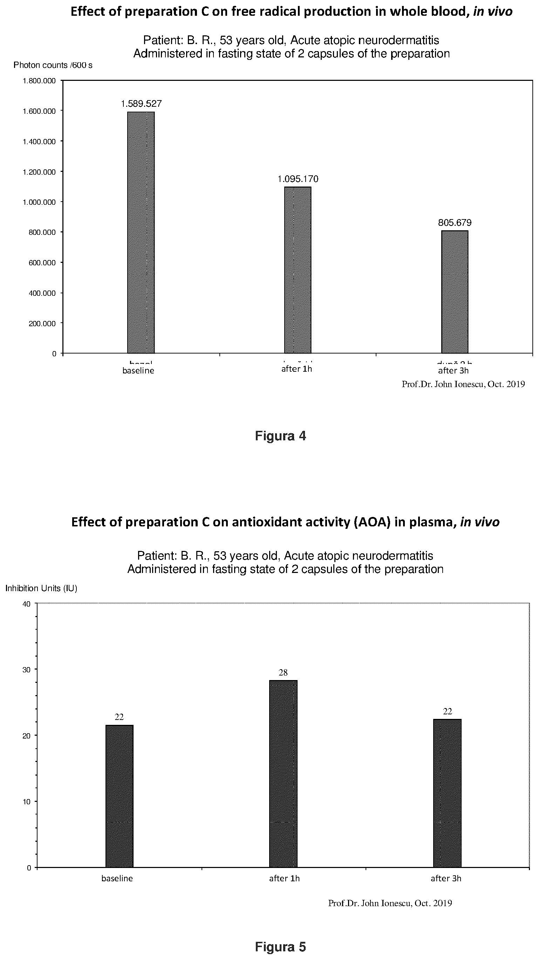

The results of the first assay (a) show a significant decrease of free radicals compared to the baseline value of 1.589.527 photons count, down to 1.095.170 photons count at one hour after administration and respectively down to 805.679 photons count at 3 hours after administration of the preparation (figure 4). The patient reported noticeable decrease of skin itching at 4 hours after administration of the two capsules of preparation C.

In the second assay (b), the overall antioxidant activity (AOA) in the patient's blood plasma was measured, collected in fasting state (baseline), at one hour and, respectively, at 3 hours after administration of two capsules of preparation C. The results of this assay prove a significant increase of AOA at one hour after administration of the preparation, followed by returning back to the baseline value after 3 hours (figure 5).

The results show that the oral administration of combination C leads to a fast increase of the overall antioxidant reserve in one hour, followed by returning back to the baseline value after 3 hours, through consumption due to the acute systemic inflammation, which proves the need for continuing the treatment.

Administration *in vivo* of the recommended dose (2 capsules/day, one in fasting state and one in the evening) for an average period of time was followed by assessing the production of free radicals in the blood, by measuring released photons in a chemiluminescence test performed in fasting state in the first day, and then after 2 days and then after 5 days.

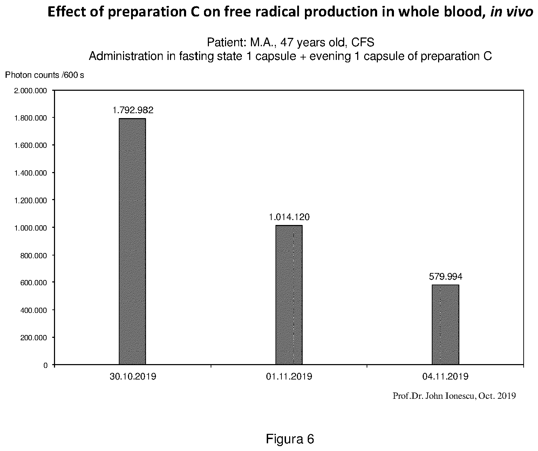

Figure 6 highlights the effect of the usual dose of combination C on the production of free radicals after 3 days of administration in a patient with chronic fatigue syndrome (CFS), recurrent viral infections and fibromyalgia.
After 3 days of treatment, the patient reported significantly reduced headache, with a reduction of foggy brain symptom and increased energy in the morning after a night sleep. This confirms the fast anti-inflammatory action of combination C, with none of the typical secondary effects associated with synthetic anti-inflammatory drugs.
The food supplement according to the invention has a wide range of practical applications in human oxidative stress related pathology (e.g. neurodegenerative diseases, cardiovascular diseases, autoimmune diseases, rheumatic diseases, diabetes, environmental diseases related to chronic accumulation of organo-toxins and heavy metals).

## Claims

1. Food supplement used to control oxidative stress and inflammation in human biological systems **characterized in that** it is a mixture which comprises three or more of the following ingredients: carnosine, coenzyme Q10, phospholipids, pyrroloquinoline quinone (PQQ), fulvic acid, oligo-elements, vitamins.

2. Food supplement according to claim 1 **characterized in that** it is a mixture wherein the phospholipids are phosphatidylcholine.

3. Food supplement according to claim 1 **characterized in that** it is a mixture wherein the oligo-elements are one or more of the followings: potassium, magnesium or phosphorus, in the form of salts compatible with the other ingredients.

4. Food supplement according to claim 1 **characterized in that** it is a mixture wherein the vitamins are one or more of the followings: folic acid and vitamin B12.

5. Food supplement according to claim 1 **characterized in that** it is a mixture which comprises coenzyme Q10 1-80%, PQQ 1-50%, and fulvic acid 1-50% and preferably it comprises coenzyme Q10 5-50%, PQQ 1-30%, and fulvic acid 2-20%, the percentage values being expressed in weight.

6. Food supplement according to claims 1, 2 and 3 **characterized in that** it is a mixture which comprises L-carnosine 10-70%, magnesium phosphate 5-50%, potassium phosphate 5-50%, coenzyme Q10 2-60%, phosphatidylcholine 2-50%, and PQQ 0.3-30.0%, and preferably it comprises L-carnosine 15-45%, magnesium phosphate 10-30%, potassium phosphate 10-30%, coenzyme Q10 10-30%, phosphatidylcholine 2-20%, and PQQ 0.5-15%, and more preferably it comprises L-carnosine 25-27%, magnesium phosphate 16-21%, potassium phosphate 16-21%, coenzyme Q10 12-17%, phosphatidylcholine 3-8%, PQQ 0.8-6%, the percentage values being expressed in weight.

7. Food supplement according to claims 1, 2, 3 and 4 **characterized in that** it is a mixture which comprises L-carnosine 10-70%, magnesium phosphate 5-50%, potassium phosphate 5-50%, coenzyme Q10 2-60%, phosphatidylcholine 2-50%, silicon
dioxide 1.5-10,0%; PQQ 0.3-40,0%, fulvic acid 0.02-15,0%, folic acid 0.01-0.5%, and vitamin B12 0.01-0.5%, and preferably it comprises L-carnosine 15-45%, magnesium phosphate 10-30%, potassium phosphate 10-30%, coenzyme Q10 10-30%, phosphatidylcholine 2-20%, silicone dioxide 5-10.0%, PQQ 0.5-15%, fulvic acid 0.2- 10%, folic acid 0.1-0.3%, and vitamin B12 0.1-0.3%, and more preferably it comprises L-carnosine 25-27%, magnesium phosphate 16-18%, potassium phosphate 16-18%, coenzyme Q10 12-14%, phosphatidylcholine 3-5%, silicone dioxide 12-14%, PQQ 0.8-2.5%, fulvic acid 0.2-1.1%, folic acid 0.14-0.2%, vitamin B12 0.14-0.2%, the percentage values being expressed in weight.

8. Food supplement according to any of the claims 1 to 7, **characterized in that** the mixture is made for oral administration in the form of tablets, capsules, powder, or for intravenous administration in the form of infusion solutions which contain single dose for the administration once or several times per day.
